# EUROPEAN PATENT APPLICATION

(11) **EP 2 684 469 A1**
(43) Date of publication of application: **15.01.2014**
(21) Application number: 12176300.7
(22) Date of filing: 13.07.2012
(51) Int. Cl.: A23L 1/30, A61K 35/74

(54) **Methods for strengthening and assessment of the natural defence of the colon against C. difficile overgrowth**

(71) Applicant: Nederlandse Organisatie voor Toegepast -Natuurwetenschappelijk Onderzoek TNO, 2628 VK Delft (NL)
(72) Inventor: Brunner, Jorg, 2628 VK Delft (NL); Keijser, Bart Jan Frederik, 2628 VK Delft (NL); Schuren, Frank Henri Johan, 2628 VK Delft (NL); Montijn, Roy Christiaan, 2628 VK Delft (NL)
(74) Representative: Jansen, Cornelis Marinus

(57) **Abstract**

The present invention is related to a new probiotic composition or food product wherein such a composition or product comprises *Clostridim bifermentans.* Such a composition or product can be advantageously used to prevent or treat a gut infection with *Clostridium difficile.*

Also part of the invention is formed by methods for assaying for *C*. *bifermentans,* for establishing the infective state of a subject by assaying for the presence of *C*. *bifermentans,* and by predicting the risk of overgrowth of *C. difficile* resulting from an antibiotic treatment before start of that treatment.

## Description

### FIELD OF THE INVENTION

The invention relates to the fields of food, medicine, bacteriology and health science. More particular, the invention relates to the field of bacterial diagnostics and bacterial-based healthy foods.

### BACKGROUND OF THE INVENTION

Probiotics, are defined by the FAO and the WHO as living microorganisms which, when administered in adequate amounts, confer a beneficial health effect on the host. Addition of these probiotic bacteria to the diet is an approach for increasing the health of the individual human or animal by promoting the presence of beneficial bacteria in the intestine. Basically probiotics act on three different levels in the intestine; they influence the microbial environment (microbe-microbe interaction), they influence the gut barrier function (microbe-gut epithelium interaction) and they are able to modulate the immune system (microbe-immune interaction).

The roles, which probiotics have on microbe-microbe interactions include among others competition for nutrients and colonization areas with harmful microorganisms including pathogenic bacteria, such as *Clostridium difficile.* Probiotics can act for example by production of antimicrobial substances, such as bacteriocins and/or the generation of restrictive physiological conditions, including acidification, caused by lactic acid and other fermentations (Teitelbaum et al. 2002. Nutritional impact of pre- and probiotics as protective gastrointestinal organisms. Annual Reviews Nutrition 22:107-138).

On improving the intestinal barrier function probiotics increase mucus production and strengthen tight junctions and preserve them in structure and function. Improving barrier function is desirable since it prevents leakage of larger molecules from the gut into the system.

Probiotics also have shown to be able to modulate the immune system. Activating the immune response depends on recognition of microbe-associated molecular patterns (MAMPs) through pattern recognition receptors like Toll-like receptors. Numerous studies have shown that probiotics can increase anti-inflammatory and suppress pro-inflammatory cytokines.

Probiotics have been used as prevention and/or treatment of several disorders like diarrhoea, constipation, IBD, IBS, allergy, etc.

Use of probiotics as dietary intervention is partly based on the concept that specific strains selected from the healthy gut microbiota may have powerful anti-pathogenic and anti-inflammatory properties, and therefore may provide resistance to intestinal diseases (Isolauri et al. 2002. Probiotics: a role in the treatment of intestinal infection and inflammation? Gut 50:54-59). In monogastric animals, including humans, commensal microbiota contribute to intestinal protection against pathogens by competition for nutrients and pathogen binding sites, and/or regulation of immune response.

Many probiotic compositions are known, since early history. The oldest probiotics are components of products we now recognize as 'normal' fermented foods, such as yoghurt, tempeh and some juices and soy beverages. An overview of medically used probiotics has been given by Blandino, G. et al., 2008, Probiotics: overview of microbiological and immunological characteristics, Expert Rev. Anti Infect. Ther. 6:497-508.

*Clostridium difficile* is a bacterium capable of producing toxins that normally occurs in low numbers in the intestine, without causing any health risk. The bacterium is able to form spores and these spores are able to survive under various conditions. If a person is subjected to an antibiotic treatment, many normal gut microbiota will be killed, but the *Clostridum* spores can survive and recolonize the gut after cessation of the antimicrobial therapy. This can result in overgrowth of *C. difficile* in the gut. Also due to other circumstances (ageing, surgery) sometimes *Clostridium difficile* bacteria can outgrow and outnumber the normal gut microbiota. Such a condition can be very harmful, causing loss of appetite, diarrhoea, abdominal pain and oedema of the intestine. This can develop into pseudomembraneous colitis and in rare cases in toxic megacolon. Treatment for *C. difficile* can be difficult due to its ability as a spore to survive antibiotic treatment. Currently the best approach is via faecal transplantation, where the gut content is replaced by that of a healthy individual.

Although many beneficial probiotic compositions are already known, there is a continuing interest both from the (bio)medical as well as from the industrial perspective in the identification of additional gut microbiota with a health promoting effect.

### SUMMARY OF THE INVENTION

The invention is directed to the use of a *Clostridium bifermentans* bacterium for the therapy against or prevention of *Clostridum difficile* infection. Therefore, the invention comprises a probiotic composition comprising *Clostridium bifermentans.* In a further embodiment, the invention comprises a *C. bifermentans* composition for use in preventing or decreasing an infection with a gut bacterium, preferably *Clostridium difficile,* or the symptoms caused by such an infection.

In yet a further embodiment, the invention is related to a method for establishing the infective status of a subject by assaying the presence of *C*. *bifermentans* in a sample. In particular, this is performed in a fecal sample.

In a further embodiment, the invention pertains to a method for assaying the presence of *C*. *bifermentans,* wherein said assay is a nucleic acid based assay.

Also part of the invention is a food product comprising *C. bifermentans,* in particular wherein said food product is a dairy product.

Further part of the invention is a method for alleviating or preventing an infection with a gut bacterium, preferably *Clostridium difficile,* or the symptoms caused by such an infection, comprising the step of administering *C. bifermentans,* in particular wherein said bacterium is administered as a probiotic composition or as a food product.

Further part of the invention is a method to evaluate the risk for *C*. *difficile* overgrowth in the gut prior to antibiotic treatment

### BRIEF DESCRIPTION OF THE DRAWINGS

Figure 1. *Clostridium difficile* counts after over night incubation as measured by quantitative polymerase chain reaction. Individual fecal samples were inoculated with 1.0E+06 *C. difficile* cells and anaerobically incubated over night. In sample 1 and 7 *C.difficile* shows almost no growth, whereas in all other samples *C. difficile* outgrowth is seen. Fecal samples from individual 1 and 7 were the only samples containing *C.*
*bifermentans*.
Figure 2. Effect of addition of *C. bifermentans* to a culture of *C*. *difficile.*

### DETAILED DESCRIPTION OF THE INVENTION

### Definitions

The term "probiotic composition" as used herein refers to a composition comprising one or more probiotic organisms and one or more acceptable excipients suitable for application to a mammal. It will be appreciated that acceptable excipientswill be well known to the person skilled in the art of probiotic composition preparation. Examples of such acceptable excipients include: sugars such as sucrose, isomerized sugar, glucose, fructose, palatinose, trehalose, lactose and xylose; sugar alcohols such as sorbitol, xylitol, erythritol, lactitol, palatinol, reduced glutinous starch syrup and reduced glutinous maltose syrup; polysaccharides as maltodextrins, starches like maize starch, rice starch, potato starch and wheat starch, emulsifiers such as sucrose esters of fatty acid, glycerin esters of fatty acid and lecithin; thickeners (stabilizers) such as carrageenan, xanthan gum, guar gum, pectin and locust bean gum; acidifiers such as citric acid, lactic acid and malic acid; fruit juices such as lemon juice, orange juice and berry juice; vitamins such as vitamin A, vitamin B, vitamin C, vitamin D and vitamin E; and minerals such as calcium, iron, manganese and zinc.

The term "dysbiosis" is defined as a state in which the microbiota produces harmful effects via (a) qualitative and quantitative changes in the intestinal microbiota itself, (b) changes in their metabolic activities; and (c) changes in their local distribution.

The term "microbiota" as used herein denominates the community of commensal microorganisms that colonise the lumen and surfaces of the gastrointestinal tract, together with the food-ingested, or transient microorganisms.

The term "permeability of the gut" as used herein refers to a characteristic of the gut mucosa to transport certain compounds or molecules from the lumen of the gut to systemic circulation. In case permeability is increased, more molecules can pass the barrier and will come into the systemic circulation, from which it can be spread all over the body. In a healthy situation the gut barrier is tight and only specific molecules are able to pass. Probiotics have the function to enhance the barrier function and thus decrease the permeability of the intestinal mucosa.

### Embodiments

The inventors have found that the naturally occurring *Clostridium bifermentans* is able to suppress the growth of *Clostridium difficile* bacteria. This finding opens the avenue for a lot of applications.

A first application is the use of *C. bifermentans* as a probiotic, optionally in combination with other probiotic organisms.

The combinations referred to above may conveniently be presented for use in the form of a probiotic composition and thus probiotic compositions comprising a combination as defined above together with one or more excipients comprise a further aspect of the invention. The individual components of such combinations may be administered either sequentially or simultaneously in separate or combined probiotic compositions.

In a preferred embodiment, *C*. *bifermentans* is combined with bifidobacteria, (like *Bifidobacterium bifidum, B*. *infantis, B. lactis, B. longum*) and / or bacteria belonging to the genus *Lactobacillus* (such as *Lactobacillus acidophilus, Lactobacillus gasseri, Lactobacillus plantarum*, *Lactobacillus buchneri*, *Lactobacillus casei, Lactobacillus johnsonii*, *Lactobacillus gallinarum*, *Lactobacillus amylovorus*, *Lactobacillus brevis*, *Lactobacillus rhamnosus*, *Lactobacillus kefir, Lactobacillus paracasei*, *Lactobacillus crispatus, Lactobacillus delbrueckii* subsp. *delbrueckii, Lactobacillus delbrueckii* subsp. *bulgaricus, Lactobacillus helveticus*, *Lactobacillus zeae, Lactobacillus plantarum* and *Lactobacillus salivarius*) and / or bacteria belonging to the genus *Streptococcus* (such as *Streptococcus thermophilus* and *Streptococcus salivarius*) and / or bacteria belonging to genus *Lactococcus* (such as *Lactococcus lactis* subsp. *cremoris* and *Lactococcus lactis* subsp. *Lactis)* and / or bacteria belonging to the genus *Bacillus* (such as *Bacillus subtilis)* and /or yeast belonging to the genus *Saccharomyces, Torulaspora* and *Candida* (such as *Saccharomyces cerevisiae*, *Torulaspora delbrueckii* and *Candida kefyr*).

It is preferred that *C*. *bifermentans* is administered in a suitable probiotic composition comprising a probiotic culture having preferably a probiotic count of between 10⁶ and 10¹¹ cfu/ml or cfu/g and a carrier medium. Compositions of the invention may be prepared by admixture, suitably at ambient temperature and atmospheric pressure and preferably under anaerobic conditions. Usually said compositions are adapted for oral administration, such as the administration forms discussed below.

In general, it can be said that administration of *C. bifermentans* decreases dysbiosis.

Furthermore, it is also contemplated that also parts of the *C*. *bifermentans* bacterium (specific proteins formed in or excreted by *C. bifermentans*" specific binding molecules on the surface of the *C. bifermentans* bacteria, specific carbohydrates and so on) can be used for exerting the effects as specified in the current invention.

In another embodiment, the invention comprises administration of *C. bifermentans* to an individual suffering from a *C*. *difficile* infection or having the risk of developing such an infection.

Therefore, said *C*. *bifermentans* according the invention can advantageously be used in a treatment comprising both cure or inhibition of a disease and prevention of a disease.

Accordingly, *C. bifermentans* or a probiotic composition comprising *C. bifermentans* may be administered to diseased subjects, but also to healthy subjects. Thus, *C. bifermentans* or a probiotic composition comprising *C. bifermentans* can advantageously be used in the treatment, inhibition or prevention of a disease, disorder or syndrome, preferably in the treatment, treatment-sparing or prevention of disease, disorder or syndrome related to *C. difficile* infection, such as loss of appetite, diarrhoea, abdominal pain, oedema of the intestine, pseudomembraneous colitis and in rare cases in toxic megacolon. Target groups for prophylactic treatment with *C. bifermentans* are healthy subjects, but especially subjects that are prone to infection, such as subjects that have or just have had antibiotic treatment, especially treatment with cephalosporins, or subject that are old or have just undergone surgery. Subjects are mammals, but especially humans, live stock, such as cattle, horses and sheep, and pet animals.

The invention further provides a method of treatment and/or prophylaxis of *C. difficile* infection, in a human or animal subject, which comprises administering to the subject a therapeutically effective amount of *C. bifermentans*, which may be used in combination with other probiotic organisms or with therapeutic agents, for example, other medicaments known to be useful in the treatment and/or prophylaxis of bacterial infections or the symptoms thereof (e.g. diarrhoea).

Thus, as a further aspect of the invention, there is provided a combination comprising said *C*. *bifermentans* together with one or more further agents.

The compositions may be in the form of tablets, capsules, oral liquid preparations, conventional food products, powders, granules, lozenges, reconstitutable powders or suspensions or oral liquid preparations.

Tablets and capsules for oral administration may be in unit dose form, and may contain one or more conventional excipients, such as binding agents, fillers, tabletting lubricants, disintegrants, and acceptable wetting agents. The tablets may be coated according to methods well known in pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and if desired, conventional flavourings or colorants. Preferably, said liquid preparation is suitable for anaerobic bacteria.

In one embodiment, the composition of the invention can be formulated as a conventional food product, more preferably, a dairy based product (e.g. fermented milk, vegetable milk, soybean milk, butter, cheese or yoghurt) or fruit juice. The composition is preferably formulated as a food or drink for adult and infant humans and animals. In an alternatively preferred embodiment, the composition is formulated as a lyophilised or spray-dried powder.

The invention also provides a method for detecting *C. bifermentans*, Detection techniques based on the detection of specific sequences are well known in the art. It is preferred that sensitive PCR techniques are used, using specific primers which enable specific amplification of *C*. *bifermentans* nucleotides. Therefore, the invention provides a kit comprising said primers, usually consisting of a first and a second primer, alternatively indicated as forward and reverse primer. These primers may be of DNA, RNA or synthetic oligonucleotides.

In some embodiments, said kit may further comprise a buffer and/or an enzyme.

The present invention therefore provides a method for evaluating the infective status of an individual by determining whether a sample of said individual comprises a *C. bifermentans* bacterium and/or a nucleic acid sequence thereof. The infective status as mentioned herein is defined as the status of infection with a gut bacterium, such as *C*. *difficile,* or the preventive status, i.e. the chance of becoming infected with a gut bacterium. Therefore, preferably, it is determined whether a sample of said individual comprises (sufficient) *C. bifermentans* bacteria. In this way, the invention provides a solution to the difficulty of determining an infective status, particularly in the determination of gastrointestinal-related risks (such as the risk of developing pseudomembraneous colitis. The presence of *C. bifermentans* in an individual, preferably in the gastrointestinal tract of an individual, indicates that said individual is of a lower risk of obtaining or suffering from an infective gastrointestinal disorder, and/or that the individual has better chances of recovery from the disorder, as compared to individuals that do not comprise *C*. *bifermentans.*

A sample as used in the invention comprises any sample containing biological material. A sample is preferably a sample of an animal. An animal as used in the invention comprises any animal with a digestive tract. Said animal preferably comprises a vertebrate, more preferably a mammal, such as for instance a human or a domestic animal kept for company or for production purposes. Non-limiting examples of animals kept for production purposes are cows, pigs and poultry. In one preferred embodiment an animal of the invention comprises a human individual. The terms animal and individual as used herein, are interchangeable.

Since the amount of nucleic acid of a *C. bifermentans* bacterium in a sample of an animal is related to the amount of *C. bifermentans* bacteria present in said animal, the amount of *C. bifermentans* -derived nucleic acid in a sample is indicative for an infective status of an animal. For instance, an elevated amount of nucleic acid of a *C*. *bifermentans* bacterium in a sample is positively correlated to an improved infective status of an individual. Therefore, the invention in a further embodiment provides a method according to the invention for determining the infective status of an animal, further comprising quantifying the amount of detected nucleic acid. An amount of nucleic acid is determined in absolute or relative quantities. A quantity is for instance compared with at least one reference value of a comparable healthy animal, with at least one reference value obtained from at least one sample with a known amount of *C*. *bifermentans*, and/or *C***.** *bifermentans* derived nucleic acid, and/or with at least one reference value derived from at least one animal with a specific disease. Reference values are for instance plotted to give a reference curve. Determining an amount of nucleic acid of the invention is for example a good method for monitoring an individual patient. An infective status of a patient can be followed over time by repeatedly determining the amount of *C*. *bifermentans* -derived nucleic acids in a sample of said patient. If the amount of *C. bifermentans* bacteria decreases, it indicates that the infective status of an individual declines, and thus that the risk increases that the individual develops a disorder caused by the infection and/or that the disorder of the individual aggravates. And in the opposite case, when an amount of a nucleic acid of a *C*. *bifermentans* bacterium in consecutive samples of an individual increases, it indicates that the infective status of an individual improves. Therefore the risk of developing a disorder and/or developing complications decreases. In this way, a decline or recovery of an infective status is assessed. In this embodiment, the risk for an individual of getting infected by a gut pathogen, especially *C*. *difficile,* is evaluated with a method according to the invention.

As the *C*. *bifermentans* bacterium mainly resides in the intestines, a sample is preferably derived from the intestines. In one embodiment of the invention, the invention therefore provides a method for determining the infective status of an animal according to the invention, wherein said sample is a fecal sample and/or if the sample comprises contents of an intestine. A sample in one embodiment comprises contents of an intestine of the animal as such a sample directly demonstrates the presence of the *C*. *bifermentans* bacterium in the intestines. Alternatively, a sample for instance comprises ileostoma effluent. Such samples are preferably derived from human patients with a stoma in the ileum. A sample further for example comprises contents of an intestine obtained during a biopsy or surgery. In order to determine the infective status of an animal, *C*. *bifermentans* is in a preferred embodiment of the invention detected by means of a nucleic acid based assay. Therefore, the invention provides a method for determining the infective status of an animal according to the invention, wherein said detecting comprises providing a primer and/or a probe according to the invention to the nucleic acid of said sample, and determining whether said primer and/or a probe specifically hybridizes to a nucleic acid of said sample. In one embodiment said primer and/or probe is administered to said sample without prior purification procedures. Preferably however, nucleic acid of a sample is firstly at least in part isolated. For instance, nucleic acid is at least in part isolated using a chaotropic agent.

Further, the invention comprises the use of administration of *C. bifermentans* to an individual when it was found according to the above described methods that said individual had a lowered infective status, to alleviate said infective status.

Next, the invention also comprises a method to evaluate the risk for *C. difficile* overgrowth prior to antibiotic treatment. In such a method the risk is predicted on basis of the presence of *C. bifermentans* next to the presence of *C. difficile* in the gut. For this detection of the presence of both baterial species a sample of the faeces is taken and the total amount of bacteria and the respective contributions of *C. difficile* and *C. bifermentans* are determined, e.g. by use of a nucleic acid based assay (such as has been performed in Example 1). The higher the amount of *C. bifermans* in comparison to the amount of *C. difficile,* the less will the risk of overgrowth of *C. difficile* be as a result of an antibiotic treatment. It will be clear that a reduction of the risk is obtained by either a (relatively) low amount of *C. difficile* and/or a (relatively) large amount of *C. bifermentans* in the fecal sample. Hence on the information on the levels of these two species of bacteria in the gut a prediction of the risk can be given.

The invention is further illustrated by the following examples. The examples are not to be interpreted as limiting the scope of the invention in any way.

### EXAMPLES

### Example 1:

### Natural resilience for Clostridium difficile outgrowth in the presence of C. bifermentans.

In the example, fecal material was obtained from seven healthy Western individuals. Fecal material was used for the inoculation of batch fermentation.

The fermentation was carried out in Standard Ileal Efflux Medium (SIEM), modified from Gibson et al., (Gibson et al., 1988), which simulates the composition of material passing the human ileo-ceacal valve, at the junction of the ileum and colon. SIEM contains per liter: 0.048 g pectin, 0.048 g xylan, 0.048 g arabinogalactan, 0.048 gamylopectin, 24 g casein, 0.4 g starch, 17.3 g Tween 80, 24 g bactopepton, 0.4 g ox-bile. 0.4 g K₂HPO₄.3H₂O, 0.72 g NaCl, 0.8 mg FeSO₄.7H₂O, 4g MgSO₄.7H₂O, 0.072 g CaCl₂.2H₂O, and 0.16 g cysteine-HCl, plus 0.8 ml of a vitamin mixture containing (per litre): 1 mg menadione, 2 mg D-biotin, 0.5 mg vitamin B12, 10 mg pantothenate, 5 mg nicotinamide, 5 mg p-aminobenzoic acid and 4 mg thiamine. The medium was buffered at pH 5.8 with 0.1 M MES.

Following pre incubation of the fecal fermentation during 4 hours under anaerobic conditions at 37C, individual samples were 'infected' with 1.0E+06 *C. difficile* cells and incubated anaerobically at 37°C during 18 hours. Next, samples were taken and DNA was isolated. For each fecal fermentation a 100 µ1 sample was transferred to a sterile screw-cap Eppendorf tube containing 0.25 ml lysis buffer (AGOWA mag Mini DNA Isolation Kit, catalogue 40410, .AGOWA, Berlin, Germany). Then 0.3 g zirconium beads (diameter, 0.1 mm, catalogue 11079101z, Biospec Products, Bartlesville, OK 74005. USA) and 0.2 ml phenol (Phenol solution BioUltra, TE-saturated, catalogue P4557, Sigma-Aldrich, St. Louis, MO, USA) were added to each sample. The samples were homogenized with a Mini-beadbeater (Mini-beadbeater 16, catalogue 607EUR, Biospec Products, Bartlesville, OK 74005. USA) for 2 min. The released DNA was purified with the AGOWA mag Mini DNA Isolation Kit according to the manufacturer's recommendations. Following, the DNA for each sample was eluted in a total volume of 40µ1 milliQ. The integrity of the DNA was inspected by agar gel electrophoresis. DNA was quantified on the NanoDrop spectrophotometer (Thermo Scientific NanoDrop 1000 Spectrophotometer, Thermo Scienific,.Wilmington, DE 19810 USA).

### Real time PCR for bacterial DNA

The total bacterial load and levels of *C. difficile* and *C. bifermentans* were established by quantitative PCR. The primer-probe set targeting the bacterial 16S rDNA gene comprised of forward primer16S-F1 (5'-CGA AAG CGT GGG GAG CAA A -3'), reverse primer 16S-R1 (5'-GTT CGT ACT CCC CAG GCG G-3') and probe 16S-P1 (FAM- ATT AGA TAC CCT GGT AGT CCA -MGB). The levels of *C. bifermentans* were analysed using the following oligonucleotide mixture: 16S-Cl-F1 (5'-GAAGTGAAAGGCTACGGCTCAA-3'), 16S-Cl-F2 (5'-CGTAAAGGGTGCGTAGGTGGTT-3') 16S-Cl-R (5'-CTCCTCTCCTGCACTCAAGTTCTC-3') and probe 16S-Cl-bif (5'- FAM-CGTAGTAAGCTTTTGAAACT-MGB -3'). The levels of *C difficile* were analysed using the following oligonucleotide mixture: 16S-Cldif-F (5'-GCAACGCGAAGAACCTTACCTA-3'), 16S-Cldif-R (5'-GAAGGGAACTCTCCGATTAAGGA-3'), and probe 16S-Cldif (5'- VIC-TGACATCCCAATGACA-MGB).

The PCR mixture consisted of 15 µ1 of 2x master mix (Universal Mastermix,catalogue GMO-UN-A100, Europe Diagenode sa, Liège, Belgium ), 1 µl of each primer (10 µM), 1 µl of the probe (5 µM), 9.5 µl DNA free water and 2.5 µl of template DNA. Amplifications were performed using a 7500 Fast Real-Time PCR System (Applied Biosystems, catalogue 4351107, Foster City, CA 94404 USA) under the following conditions: 2 min at 50°C and 10 min at 95°C, followed by 45 cycles of 15 s at 95°C and 1 min at 60°C.

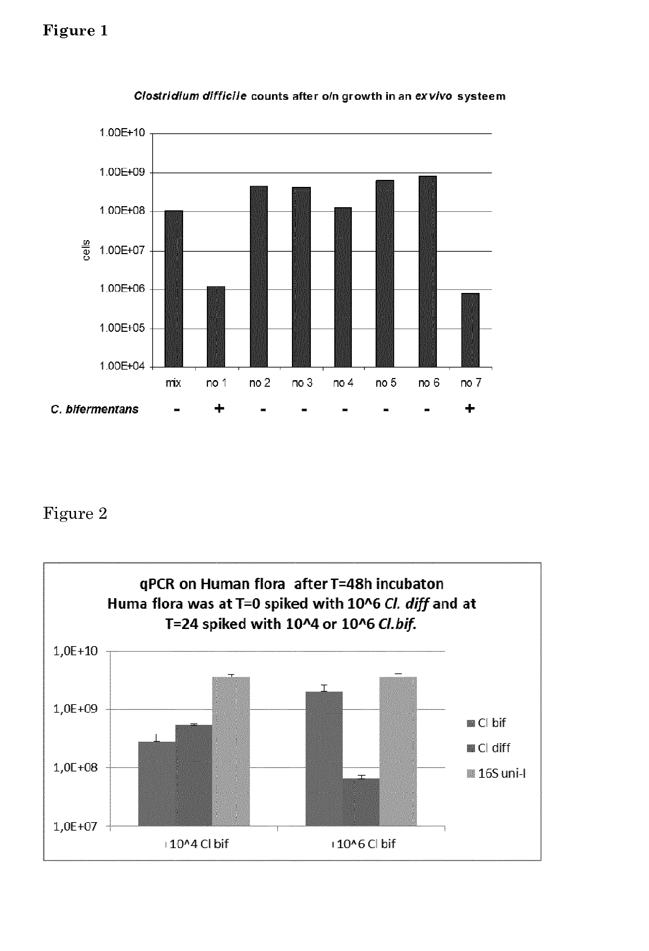

In this experiment, it was found that in those samples where *C. bifermentans* was naturally present in the fecal inocula, *C. difficile* did not grow during the overnight incubations. Contrastingly, in those samples where *C, bifermentans* was absent, *C. difficile* proliferated 100 fold to levels <10^8 cells per ml (see Fig. 1)

### Example 2

### Limiting the outgrowth of C. difficile by adding C. bifermentans to a fecal sample.

Human fecal material (10⁸ CFU/ml) was spiked with *Clostridium difficile* (ATCC 9689) at 10⁶ CFU/ml and was cultivated in a batch fermentor under anaerobic conditions for 24 hours in Standard Ileal Efflux Medium (see Example 1). Next, the culture was diluted 1:1000 resulting in a density of 10⁸ CFU/ml and *Clostridium bifermentans* (ATCC 638) was added at a level of 10⁶ or 10⁴ CFU/ml. Again the fermentor was incubated under anaerobic conditions at 37°C for the following 24 hour. After this incubation, samples were taken, DNA was extracted and was tested for the presence of *C. difficile* and *C. bifermentans* according to the method described in full in Example 1. From this experiment it became apparent (see Fig. 2) that inoculation of *C. bifermentans* at low levels (10⁴) resulted in the outgrowth of *C. difficile* to a level of 10⁸-10⁹ CFU per ml. However, when *C. bifermentans* was inoculated at a level of 10⁶ CFU/ml, *C. dificile* remained at a level 1 log order lower. No effects were observed on the total bacterial load.

## Claims

1. Probiotic composition comprising *Clostridium bifermentans*

2. *C. bifermentans* composition for use in preventing or decreasing an infection with a gut bacterium, preferably *Clostridium difficile,* or the symptoms caused by such an infection.

3. Method for establishing the infective status of a subject by assaying the presence of *C*. *bifermentans* in a sample.

4. Method according to claim 3, wherein said sample is a fecal sample.

5. Method for assaying the presence of *C*. *bifermentans*, wherein said assay is a nucleic acid based assay.

6. Food product comprising *C*. *bifermentans*.

7. Food product according to claim 6, wherein said food product is a dairy product.

8. Method for alleviating or preventing an infection with a gut bacterium, preferably *Clostridium difficile,* or the symptoms caused by such an infection, comprising the step of administering *C. bifermentans*.

9. Method to evaluate the risk for C. difficile overgrowth in the gut prior to antibiotic treatment


SEQUENCE LISTING

<110> Nederlandse Organisatie voor
      toegepast-natuurwetenschappelijk onderzoek TNO

<120> Methods for strengthening and assessment of the natural defence
      of the colon against C. difficile overgrowth.

<130> P97069EP00

<160> 10

<170> PatentIn version 3.3

<210> 1
<211> 19
<212> DNA
<213> Artificial

<220>
<223> primer 16S-F1

<400> 1
cgaaagcgtg gggagcaaa                                                    19


<210> 2
<211> 19
<212> DNA
<213> Artificial

<220>
<223> primer 16S-R1

<400> 2
gttcgtactc cccaggcgg                                                    19


<210> 3
<211> 21
<212> DNA
<213> Artificial

<220>
<223> probe 16S-P1

<400> 3
attagatacc ctggtagtcc a                                                 21


<210> 4
<211> 22
<212> DNA
<213> Artificial

<220>
<223> primer 16S-Cl-F1

<400> 4
gaagtgaaag gctacggctc aa                                                22


<210> 5
<211> 22
<212> DNA

<213>  Artificial

<220>
<223>  primer 16S-Cl-F2

<400>  5
cgtaaagggt gcgtaggtgg tt                                                22


<210>  6
<211>  24
<212>  DNA
<213>  Artificial

<220>
<223>  primer 16S-Cl-R1

<400>  6
ctcctctcct gcactcaagt tctc                                              24


<210>  7
<211>  20
<212>  DNA
<213>  Artificial

<220>
<223>  probe 16S-Cl--bif

<400>  7
cgtagtaagc ttttgaaact                                                   20


<210>  8
<211>  22
<212>  DNA
<213>  Artificial

<220>
<223>  primer 16S-Cldif-F

<400>  8
gcaacgcgaa gaaccttacc ta                                                22


<210>  9
<211>  23
<212>  DNA
<213>  Artificial

<220>
<223>  primer 16S-Cldif-R

<400>  9
gaagggaact ctccgattaa gga                                               23


<210>  10
<211>  16
<212>  DNA
<213>  Artificial

<220>
<223>  probe 16S-Cldif

<400>  10
tgacatccca atgaca                                                        16